# EUROPEAN PATENT APPLICATION

(11) **EP 4 317 149 A1**
(43) Date of publication of application: **07.02.2024**
(21) Application number: 22799147.8
(22) Date of filing: 06.05.2022
(51) Int. Cl.: C07D 403/06, A61K 31/5377, A61P 3/04, A61P 3/10, A61P 29/00, A61P 15/10

(54) **CRYSTALLINE FORM V OF MELANOCORTIN RECEPTOR AGONIST COMPOUND, AND METHOD FOR PREPARING SAME**

(30) Priority: 06.05.2021 KR 20210058701
(71) Applicant: LG Chem, Ltd., Seoul 07336 (KR)
(72) Inventor: HAM, Jin Ok, Daejeon 34122 (KR); LEE, Ho Yeon, Daejeon 34122 (KR); LEE, Sang Dae, Daejeon 34122 (KR); KIM, Ji Yoon, Daejeon 34122 (KR); PARK, Jong Won, Daejeon 34122 (KR); KIM, Sung Won, Daejeon 34122 (KR); CHUN, Seul Ah, Daejeon 34122 (KR)
(74) Representative: Hoffmann Eitle
(86) International application number: PCT/KR2022/006478
(87) International publication number: WO 2022/235104

(57) **Abstract**

The present invention relates to crystalline form V of a compound represented by chemical formula 1, a method for preparing same, and a pharmaceutical composition comprising same. Crystalline form V of the compound represented by chemical formula 1 according to the present invention may be characterized by an XRD pattern, a DSC profile, and/or a TGA profile.

## Description

### TECHNICAL FIELD

### [Cross-reference to Related Application]

The present invention claims the benefit of priority based on Korean Patent Application No. 10-2021-0058701, filed on May 6, 2021,

the entire disclosure of which is incorporated as part of the specification.

### [Technical Field]

The present invention relates to a crystalline form V of a novel compound exhibiting an excellent agonistic activity for a melanocortin receptor, a method for preparing the same, and a pharmaceutical composition comprising the same.

### BACKGROUND ART

Leptin protein is a hormone secreted by adipocytes, and its secretion amount increases with an increase in body fat content. It regulates functions of various neuropeptides produced from hypothalamus, thereby regulating various in vivo functions, including appetite, body fat content, and energy metabolism (Schwartz, et al., Nature 404, 661-671 (2000)). The leptin protein signal transduction for controlling appetite and body weight is made through the regulation of many factors downstream, the most representative of which are melanocortin, agouti-related peptide (AgRP), and neuropeptide Y (NPY) hormones.

When the concentration of leptin in the blood increases as a result of excess calories in vivo, the secretion of proopiomelanocortin (POMC) protein hormone from the pituitary gland increases and the production of AgRP and NPY decreases. A small peptide hormone, alpha-melanocyte-stimulating hormone (MSH), is produced from POMC neurons. The hormone is an agonist for melanocortin-4 receptors (MC4R) of second-order neurons and ultimately induces appetite decrease. Meanwhile, when the concentration of leptin decreases as a result of calorie deficiency, the expression of AgRP, an MC4R antagonist, increases, and the expression of NPY also increases, which ultimately promotes appetite. That is, according to the change of leptin, the alpha-MSH hormone and the AgRP hormone act as agonists and antagonists for MC4R and thus are involved in appetite control.

The Alpha-MSH hormone binds to three MCR subtypes in addition to MC4R to induce various physiological reactions. Five MCR subtypes have been identified so far. Among them, MC1R is expressed mainly in skin cells and is involved in regulating melanin pigmentation (skin pigmentation). MC2R is expressed mainly in the adrenal gland and is known to be involved in the production of glucocorticoid hormones. Its ligand is only adrenocorticotropic hormone (ACTH) derived from POMC. MC3R and MC4R, which are expressed mainly in the central nervous system, are involved in regulating appetite, energy metabolism, and body fat storage efficiency, and MC5R expressed in various tissues is known to regulate exocrine function (Wikberg, et al., Pharm Res 42 (5) 393-420 (2000)). In particular, activation of the MC4R receptor induce appetite decrease and energy metabolism increase and thus has an effect of efficiently reducing body weight. Therefore, it has been proven to be a major action point in the development of anti-obesity drugs (Review: Wikberg, Eur. J. Pharmacol 375, 295-310 (1999)); Wikberg, et al., Pharm Res 42 (5) 393-420 (2000); Douglas et al., Eur J Pharm 450, 93-109 (2002); O'Rahilly et al., Nature Med 10, 351-352 (2004)).

The role of MC4R in the control of appetite and body weight was primarily demonstrated through an experiment in an animal model of abnormal expression of the agouti protein (agouti mouse). In the case of the Agouti mouse, it was found that due to genetic mutation, the agouti protein was expressed at a high concentration in the central nervous system and acted as an antagonist of MC4R in the hypothalamus to cause obesity (Yen, TT et al., FASEB J. 8, 479-488 (1994); Lu D., et al. Nature 371, 799-802 (1994)). Subsequent research results showed that the agouti-related peptides (AgRP) similar to the actual agouti protein were expressed in hypothalamic nerves, and these are also known to be antagonists for MC4R and be involved in controlling appetite (Shutter, et al., Genes Dev., 11, 593-602 (1997); Ollman, et al. Science 278, 135-138 (1997)).

Intracerebral administration of alpha-MSH, which is an in vivo MC4R agonist, to animals leads to the effect of reducing appetite. When treating the animals with SHU9119 (peptide) or HS014 (peptide), which are MC4R antagonists, it was observed that appetite increased again (Kask et al., Biochem. Biophys. Res. Comm. 245, 90-93 (1998)). In addition, in animal studies using Melanotan II (MTII, Ac-Nle-c[Asp-His-DPhe-Arg-Trp-Lys]-NH2) and the similar agonist thereof, HP228, after intracerebral, intraperitoneal, or subcutaneous administration, efficiencies of inhibiting appetite, reducing body weight, increasing energy metabolism, etc. were found. (Thiele T. E., et al. Am J Physiol 274 (1 Pt 2), R248-54 (1998); Lee M. D., et al. FASEB J 12, A552 (1998); Murphy B., et al. J Appl Physiol 89, 273-82 (2000)). On the contrary, administration of the representative SHU9119 to animals showed significant and sustained feed intake and weight gain, providing pharmacological evidence that MCR agonists could be anti-obesity agents. The effect of reducing appetite, which is clearly exhibited upon administration of MTII, was not observed in MC4R knock-out (KO) mice. This experimental result proves again that the appetite-reducing effect is achieved mainly through the activation of MC4R (Marsh, et al., Nat Genet 21, 119-122 (1999)).

Anorectic agents acting on the central nervous system were the main types of anti-obesity drugs developed so far. Among them, most were drugs that modulate the action of neurotransmitters. Examples include noradrenalin agents (phentermine and mazindol), serotonergic agents, fluoxetine and sibutramine, and the like. However, the neurotransmitter modulators have a wide range of effects on various physiological actions in addition to appetite suppression, through numerous subtype receptors. Accordingly, the modulators lack selectivity for each subtype, and thus have a major disadvantage in that they are accompanied by various side effects when administered for a long period.

Meanwhile, melanocortin agonists are neuropeptides, not neurotransmitters. Given that in MC4R gene KO mice, all functions other than energy metabolism are normal, they have an advantage as an action point in that they can induce only weight loss through appetite suppression without affecting other physiological functions. In particular, the receptor is a G-protein coupled receptor (GPCR) that belongs to the most successful category of new drug action points developed so far. Thus, the action point greatly differ from existing action points in that it is relatively easy to secure selectivity for subtype receptors.

As an example of utilizing a melanocortin receptor as an action point, international publication nos. WO 2008/007930 and WO 2010/056022 disclose compounds as agonists of the melanocortin receptor.

In addition, the inventors of the present invention have conducted extensive studies and invented a novel compound of the following formula 1 having an excellent agonistic activity selective for a melanocortin receptor, in particular, melanocortin-4 receptor (MC4R), and a method for preparing the same (application no. KR 10-2019-0141649 (filed on 7 November 2019)) : 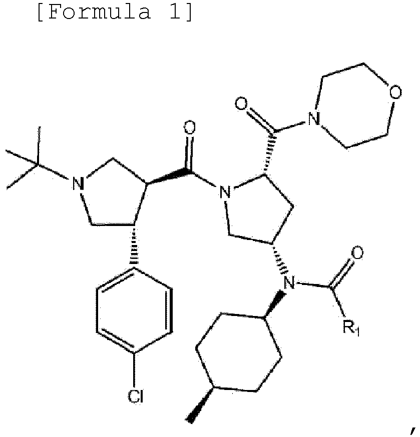 wherein R₁ is a C₂-C₅ alkyl.

Meanwhile, the crystal structure of a pharmaceutically active ingredient often affects the chemical stability of the drug. Different crystallization conditions and storage conditions can lead to changes in the crystal structure of the compound, and sometimes the accompanying production of other forms of the crystalline form. In general, an amorphous drug product does not have a regular crystal structure, and often has other defects such as poor product stability, smaller particle size, difficult filtration, easy agglomeration, and poor flowability. Thus, it is necessary to improve various physical properties of the product. However, in general, a person skilled in the art could not easily predict under what conditions a compound will crystallize effectively and which crystalline form will be advantageous in terms of reproducibility, stability, and bioavailability, and thus there is a need to study different types of various crystal structures for a single compound.

### [Prior art documents]

### [Patent Documents]

(Patent Document 1) International Patent Application Publication No. WO 2008/007930
(Patent Document 2) International Patent Application Publication No. WO 2010/056022

### DISCLOSURE OF THE INVENTION

### TECHNICAL PROBLEM

An aspect of the present invention provides a stable crystalline form of a novel compound having an excellent agonistic activity, which is selective for a melanocortin receptor, in particular, melanocortin-4 receptor (MC4R), and a method for preparing the same.

Another aspect of the present invention provides a pharmaceutical composition comprising the stable crystalline form of the novel compound.

### TECHNICAL SOLUTION

According to an aspect of the present invention,
there is provided a crystalline form V of a compound of the following formula 1, a pharmaceutically acceptable salt, or a solvate thereof,
wherein the X-ray powder diffraction pattern has 3 or more or 5 or more characteristic peaks selected from among peaks with the following diffraction angles (2Θ values) of: 4.42±0.2°, 4.92±0.2°, 7.20±0.2°, 7.84±0.2°, 9.2210.2°, 12.22±0.2°, 12.58±0.2°, 16.20±0.2°, 16.48±0.2°, 17.72±0.2°, 20.24±0.2°, 20.08±0.2°, and 23.65±0.2°, 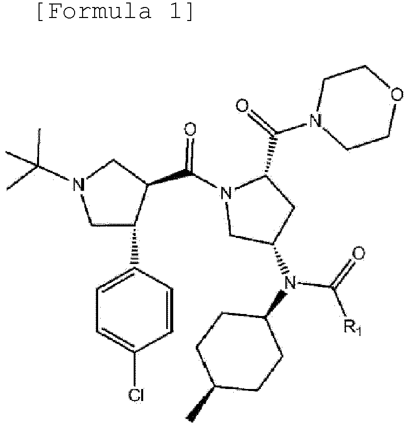 wherein
   R₁ is a C₂-C₅ alkyl.

Since the compound of formula 1 can have an asymmetric carbon center and an asymmetric axis or an asymmetric plane, it can exist as cis or trans isomers, R or S isomers, racemates, diastereomer mixtures, and individual diastereomers, all of which are within the scope of the compound of formula 1.

In the present specification, unless otherwise specified for convenience, the compound of formula 1 is used to include all of the compound of formula 1, a pharmaceutically acceptable salt, an isomer, and a solvate thereof.

In one embodiment, according to the present invention, in formula 1, R₂ is a C₂ to C₅ alkyl. In another embodiment, according to the present invention, in formula 1, R₁ is a straight-chain or branched C₂ to C₅ alkyl, for example, ethyl, n-propyl, iso-propyl, n-butyl, iso-butyl, sec-butyl, or tert-butyl.

In another embodiment, according to the present invention, in formula 1, R₁ is a C₂ or C₃ alkyl. In another embodiment, according to the present invention, in formula 1, R₁ is a straight-chain or branched C₂ or C₃ alkyl, for example, ethyl, n-propyl, or iso-propyl.

In one embodiment, according to the present invention, the pharmaceutically acceptable salt includes, but is not limited to, acid-addition salts which are formed from inorganic acids, such as hydrochloric acid, sulfuric acid, nitric acid, phosphoric acid, hydrobromic acid, and hydroiodic acid; organic carboxylic acids, such as tartaric acid, formic acid, citric acid, acetic acid, trichloroacetic acid, trifluoroacetic acid, gluconic acid, benzoic acid, lactic acid, fumaric acid, and maleic acid; or sulfonic acids, such as methanesulfonic acid, benzenesulfonic acid, p-toluenesulfonic acid, or naphthalene-sulfonic acid.

In one embodiment, according to the present invention, the solvate may include a hydrate; or a solvate with an organic solvent selected from the group consisting of methanol, ethanol, 2-propanol, 1,2-propanediol, 1,3-propanediol, n-butanol, 1,4-butanediol, tert-butanol, acetic acid, acetone, butyl acetate, methyl acetate, ethyl acetate, propyl acetate, t-butyl acetate, isobutyl acetate, methylethylketone, 2-pentanone, tetrahydrofuran, acetonitrile, chloroform, toluene, dimethyl ether, diethyl ether, methyl ethyl ether, methyl phenyl ether, ethyl phenyl ether, diphenyl ether, and mixtures of two or more thereof.

In one embodiment, according to the present invention, the crystalline form V may be a crystalline form of the pharmaceutically acceptable salt of the compound of formula 1.

The pharmaceutically acceptable salt of the compound of formula 1 may be a hydrochloride compound of the following formula 2: 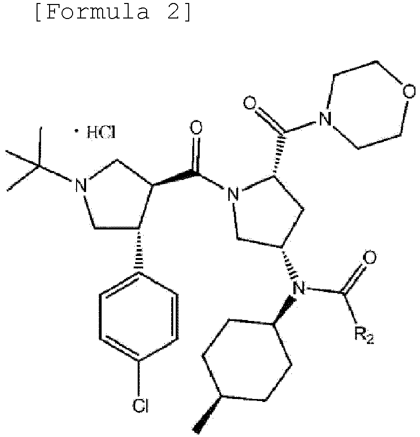 wherein R₂ is a C₂-C₅ alkyl.

In another embodiment, according to the present invention, the pharmaceutically acceptable salt of the compound of formula 1 may be *N*-((3*S*,5*S*)-1-((3*S*,4*R*)-1-(*tert-*butyl)-4-(4-chlorophenyl)pyrrolidine-3-carbonyl)-5-(morpholine-4-carbonyl)pyrrolidin-3-yl)-*N*-((1*s*,4*R*)-4-methylcyclohexyl)isobutyramide hydrochloride of the following formula 3:

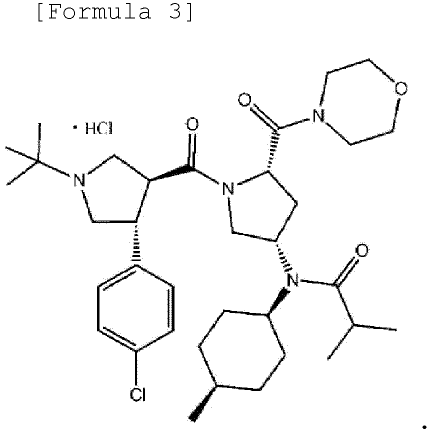

In another embodiment, according to the present invention, the crystalline form V may be a crystalline form of the solvate, specifically the hydrate of the pharmaceutically acceptable salt of the compound of formula 1.

More specifically, the crystalline form V may be a crystalline form of the solvate of the hydrochloride of the compound of formula 1.

In one embodiment, according to the present invention, the solvate of the hydrochloride of the compound of formula 1 may be a solvate with an aromatic ether.

In another embodiment, according to the present invention, the solvate of the hydrochloride of the compound of formula 1 may be a solvate with methyl phenyl ether. Methyl phenyl ether is also referred to as anisole (C₇H₈O). Accordingly, the solvate may be a solvate with anisole.

In one embodiment, according to the present invention, the crystalline form V may be a crystalline form of the compound of the following formula 4.

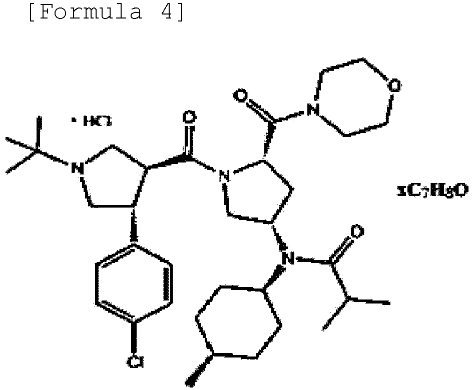

The crystalline form V according to the present invention shows characteristic peaks including 3 or more, 5 or more, or all peaks selected from among 4.42±0.2°, 4.92±0.2°, 7.20±0.2°, 7.84±0.2°, 9.22±0.2°, 12.22±0.2°, 12.58±0.2°, 16.20±0.2°, 16.48±0.2°, 17.72±0.2°, 20.24±0.2°, 20.08±0.2°, and 23.65±0.2°, as analyzed by X-ray powder diffraction (XRD).

In one embodiment, according to the present invention, the crystalline form V may show characteristic peaks including 3 or more, 5 or more, or all peaks selected from among 4.42±0.2°, 7.84±0.2°, 12.58±0.2°, 16.20±0.2°, 20.08±0.2°, and 23.65±0.2°, as analyzed by X-ray powder diffraction (XRD).

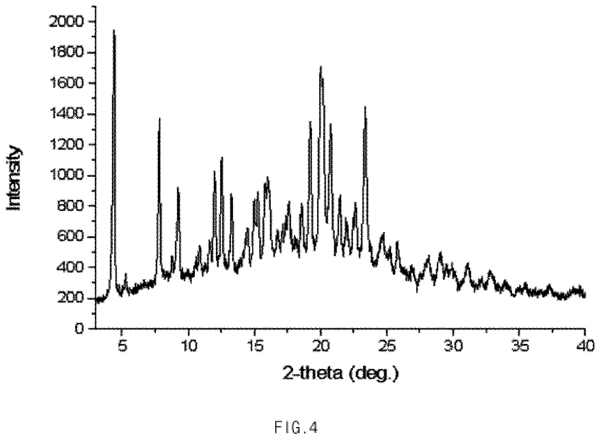

In one embodiment, according to the present invention, the crystalline form V may have the XRD pattern shown in FIG. 4.

In one embodiment, according to the present invention, the crystalline form V may have an endothermic peak at 120 to 130°C in the differential scanning calorimetry (DSC) profile.

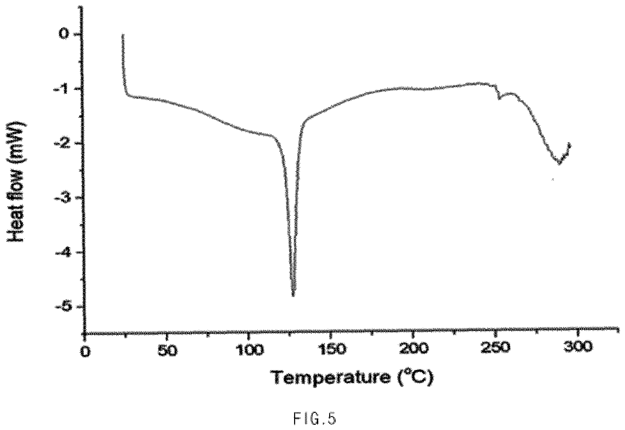

In one embodiment, according to the present invention, the crystalline form V may have the DSC profile shown in FIG. 5.

The crystalline form V according to the present invention, in the thermogravimetric analysis (TGA) profile, may have 20% or less, for example, 1% to 20%, 5% to 18%, 10% to 17%, 10% to 15%, or 11% to 13% of a weight loss when heated to a temperature of 150°C or less, for example, at a temperature of 120 to 130°C.

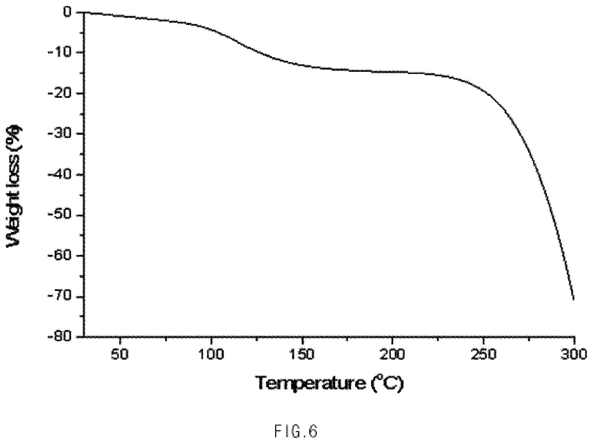

In one embodiment, according to the present invention, the crystalline form V may have the TGA profile shown in FIG. 6.

In another embodiment, according to the present invention, the crystalline form V may show characteristic peaks including 3 or more, 5 or more, 6 or more, or all peaks selected from among 4.92±0.2°, 7.20±0.2°, 9.22±0.2°, 12.22±0.2°, 16.48±0.2°, 17.72±0.2°, and 20.24±0.2°, as analyzed by X-ray powder diffraction (XRD).

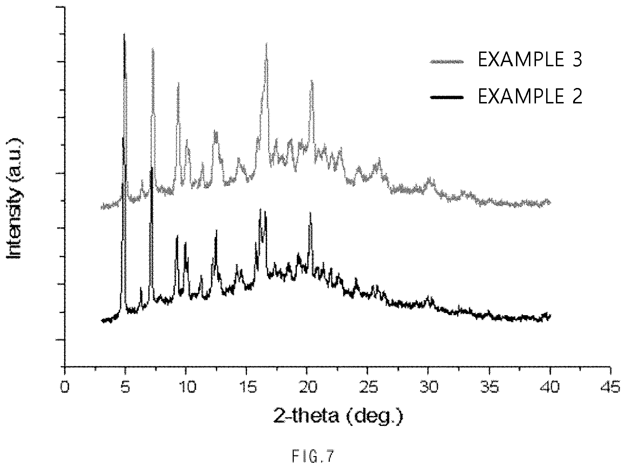

In another embodiment, according to the present invention, the crystalline form VI may have the XRD pattern shown in FIG. 7.

In another embodiment, according to the present invention, the crystalline form V may have an endothermic peak at 190 to 200°C, specifically 190 to 195°C, and more specifically 192 to 194°C in the differential scanning calorimetry (DSC) profile.

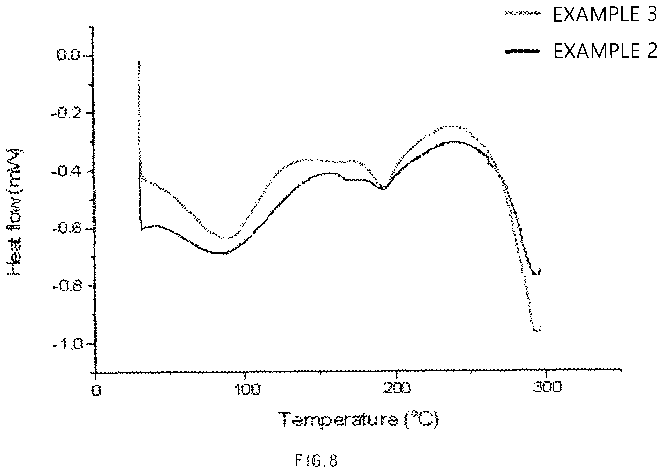

In another embodiment, according to the present invention, the crystalline form V may have the DSC profile shown in FIG. 8.

In another embodiment, according to the present invention, the crystalline form V, in the thermogravimetric analysis (TGA) profile, may have 10% or less, for example, 1% to 5% or 1% to 3% of a weight loss when heated to a temperature of 150°C or less, for example, at a temperature of 120 to 130°C.

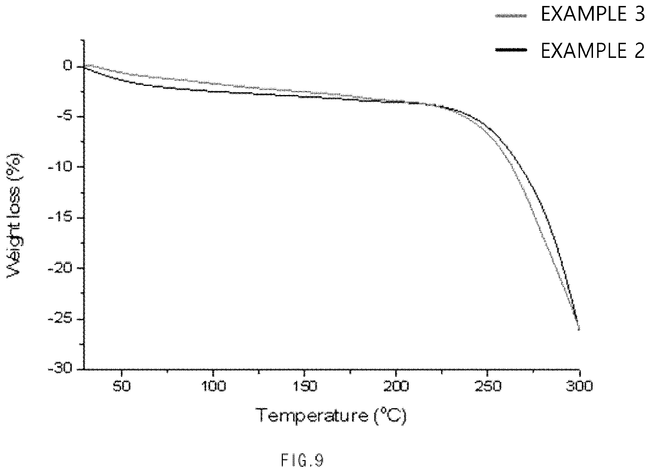

In another embodiment, according to the present invention, the crystalline form V may have the TGA profile shown in FIG. 9.

In the present specification,
X-ray diffraction (XRD) analysis shows the results obtained by PXRD (Cu Kα radiation (λ=1.54Å), Rigaku, Miniflex 600, Japan).

Differential scanning calorimetry (DSC) analysis shows the results obtained by DSC (Q2000, TA instrument, USA).

Thermogravimetric analysis (TGA) shows the results obtained by TGA (TGA8000, PerkinElmer, USA).

The crystalline form V may have higher purity and better solubility or stability than a crude compound of formula 1, an amorphous compound of formula 1, or other crystalline forms of the compound of formula 1. In particular, due to the suppressed decomposition and moisture absorption, the crystalline form V may be physically and chemically more stable, and thus may be easily used for preparing a pharmaceutical composition. In addition, the agonistic ability for the melanocortin-4 receptor and preventive or therapeutic effects on diseases, such as obesity, diabetes, inflammation, erectile dysfunction, or the like, of the crystalline form V of the compound of formula 1, may be more excellent than those of known melanocortin-4 receptor agonists. However, the effects of the present invention are not limited thereto.

In another aspect, the present invention provides a method for preparing the crystalline form V, comprising the steps of: preparing a mixed solution by dissolving the compound of formula 1 in a crystallization solvent containing an aromatic ether; and obtaining crystals from the mixed solution.

First, the compound represented by formula 1 is dissolved in a crystallization solvent containing an aromatic ether.

The compound of formula 1 for preparing the crystalline form V may be a compound of formula 1, a salt thereof, an isomer thereof, or a solvate thereof.

The compound of formula 1 may be obtained by the preparation method described in the specification of Korean patent application no. KR 10-2019-0141649 (filed on 7 November 2019) .

The crystallization solvent should be used containing an aromatic ether solvent. The 'aromatic ether' solvent may be used without particular limitation as long as it is an ether compound having an aromatic substituent.

In one embodiment of the present invention, the aromatic ether may include, but is not limited to, methyl phenyl ether, ethyl phenyl ether, diphenyl ether, or a mixture thereof.

In another embodiment of the present invention, the aromatic ether includes methyl phenyl ether (also referred to as 'anisole').

In another embodiment of the present invention, the crystallization solvent may further include an aprotic organic solvent in addition to the aromatic ether, and specifically, it may further include an aliphatic aprotic organic solvent distinct from the aromatic ether solvent.

The aliphatic aprotic organic solvent may include diethyl ether, pentane, hexane, heptane, cyclohexane, or a mixture thereof.

In one embodiment, according to the present invention, the aliphatic aprotic organic solvent may include diethyl ether (also referred to as 'ethyl ether').

In one embodiment, according to the present invention, the crystallization solvent may include an aromatic ether and an aliphatic aprotic organic solvent, specifically, anisole and ethyl ether.

In another embodiment, according to the present invention, as the crystallization solvent, the aromatic ether and the aliphatic aprotic organic solvent may be used in a volume ratio of 1:1 to 1:10, specifically 1:1 to 1:10, 1:1 to 1:5, 1:2 to 1:4, or 1:3.

In another embodiment, according to the present invention, the crystallization solvent may be a mixed solvent in which anisole and ethyl ether are mixed in a volume ratio of 1:3.

Dissolution of the compound of formula 1 may be carried out without or with stirring at a temperature of 20 to 25°C.

The crystallization solvent may be used in an amount sufficient to completely dissolve the compound of formula 1. For example, a mixed solution in which the compound of formula 1 has been dissolved may have a concentration of 10 g/mL to 200 mg/mL, 50 mg/mL to 150 mg/mL, 80 mg/mL to 120 mg/mL, or 100 mg/mL.

The method includes the step of obtaining crystals from the mixed solution. The crystals may be obtained, for example, by cooling the solution, by evaporating the solvent, by adding an antisolvent for supersaturation, or by using methods, such as slurry conversion, or the like.

In one embodiment, according to the present invention, a precipitate formed by storing the mixed solution at room temperature for a certain period of time may be filtered and washed to obtain crystals.

Storage may be carried out for 12 hours to 48 hours, such as 20 hours to 36 hours, or 24 hours at a temperature of the mixed solution of 20°C to 30°C and without stirring.

The crystalline form V as obtained above may have higher purity and better solubility or stability than the crude compound of formula 1, the amorphous compound of formula 1, or any other crystalline forms of formula 1. In particular, due to the suppressed decomposition and moisture absorption, the crystalline form V may be physically and chemically more stable, and thus may be easily used for preparing a pharmaceutical composition. However, the effects of the present invention are not limited thereto.

In one embodiment, according to the present invention, the method may further include a step for washing the obtained crystals with an aliphatic ether solvent.

Through the washing step, the purity of the obtained crystals may be increased, the yield of the finally produced compound of formula 1 may be improved, and the stability of the compound may be improved. However, the effects of the present invention are not limited thereto.

An aliphatic ether solvent is used for washing. The 'aliphatic ether' solvent may be used without particular limitation as long as it is an ether compound having an aliphatic substituent.

In one embodiment of the present invention, the aliphatic ether may include, but is not limited to, dimethyl ether, diethyl ether, methyl ethyl ether, methyl propyl ether, ethyl propyl ether, or a mixture thereof.

In another embodiment of the present invention, the aliphatic ether may include diethyl ether.

In another embodiment of the present invention, the crystallization solvent may include methyl phenyl ether and diethyl ether, and the washing solvent may include diethyl ether.

The washing step may include washing the crystals obtained first above with an aliphatic aprotic organic solvent at least once and then storing them.

In one embodiment of the present invention, after the washing, a precipitate formed by storing the crystals at, for example, 0°C to 200°C, 20°C to 25°C, 25°C to 60°C or 40°C to 80°C may be obtained by filtration.

In another embodiment of the present invention, after the washing, a precipitate formed by storing the crystals at the above temperature for 60 minutes to 60 days, for example, 60 minutes to 45 days, 2 hours to 45 days, 5 hours to 45 days, 5 hours to 30 days, 5 hours to 24 hours, 6 hours to 12 hours, 6 hours to 10 hours, 8 hours to 30 days, 10 hours to 45 days, 12 hours to 40 days, 15 days to 45 days, or 20 days to 40 days may be obtained by filtration.

During the recrystallization, storing may be carried out at the above temperature for the above period with or without stirring.

The crystalline form V as obtained above may have higher purity and better solubility or stability than the crude compound of formula 1, the amorphous compound of formula 1, or any other crystalline forms of formula 1. In particular, due to the suppressed decomposition and moisture absorption, the crystalline form V may be physically and chemically more stable, and thus may be easily used for preparing a pharmaceutical composition. However, the effects of the present invention are not limited thereto.

In another aspect, the present invention provides a pharmaceutical composition comprising: (i) the crystalline form V; and (ii) a pharmaceutically acceptable carrier.

The crystalline form V, according to the present invention, exhibits excellent agonistic actions on melanocortin receptors, in particular, melanocortin-4 receptors (MC4R). Thus, the present invention can provide a pharmaceutical composition for agonizing melanocortin receptors, the composition containing the above-described crystalline form V as an active ingredient. Specifically, the pharmaceutical composition may be a composition for agonizing the function of the melanocortin-4 receptor.

In addition, since the pharmaceutical composition can exhibit excellent effects of preventing or treating obesity, diabetes, inflammation, and erectile dysfunction, it may be a composition for preventing or treating obesity, diabetes, inflammation, or erectile dysfunction. However, the use of the present invention is not limited the diseases.

As used herein, "carrier" refers to a compound that facilitates the introduction of compounds into a cell or tissue.

When the crystalline form V of the present invention is administered for clinical purposes, the total daily dose to be administered to a host in a single dose or in divided doses may be preferably in the range of 0.01 to 10 mg/kg body weight. However, the specific dose level for an individual patient may vary depending on the specific compound to be used, the patient's weight, sex, health status, diet, administration time of the drug, administration method, excretion rate, drug combination, the severity of the disease, or the like.

The crystalline form V of the present invention may be administered by any route as desired. For example, the amorphous compound of the present invention may be administered parenterally or orally.

The pharmaceutical composition of the present invention may be in various oral dosage forms, such as tablets, pills, powders, capsules, granules, syrups, or emulsions, or parenteral dosage forms, such as injection preparations for intramuscular, intravenous, or subcutaneous administration.

Preparations for injection may be prepared, according to known techniques, using suitable dispersing agents, wetting agents, suspending agents, or excipients.

Excipients that can be used in the pharmaceutical preparation of the present invention include, but are not limited to, sweeteners, binders, solubilizers, solubilizing agents, wetting agents, emulsifiers, isotonic agents, adsorbents, disintegrants, antioxidants, preservatives, lubricants, fillers, fragrances, etc. For example, as excipients, lactose, dextrose, sucrose, mannitol, sorbitol, cellulose, glycine, silica, magnesium aluminum silicate, starch, gelatin, gum tragacanth, arginic acid, sodium alginate, methylcellulose, sodium carboxymethyl cellulose, water, ethanol, polyethylene glycol, polyvinyl pyrrolidone, sodium chloride, calcium chloride, orange essence, strawberry essence, vanilla flavor, etc. may be used.

When the pharmaceutical composition of the present invention is in an oral dosage form, examples of the carrier to be used may include, but are not limited to, cellulose, calcium silicate, corn starch, lactose, sucrose, dextrose, calcium phosphate, stearic acid, magnesium stearate, calcium stearate, gelatin, talc, etc.

When the pharmaceutical composition of the present invention is in an injectable preparation form, examples of the carrier may include, but are not limited to, water, saline, aqueous glucose solution, an aqueous sugar-like solution, alcohols, glycol, ethers, oils, fatty acids, fatty acid esters, glycerides, etc.

In another aspect, there is provided a crystalline form V as described above for use in agonizing the functions of melanocortin receptors, in particular, melanocortin-4 receptors (MC4R).

In one embodiment, there is provided a crystalline form V as described above for use in treating or preventing obesity, diabetes, inflammation, or erectile dysfunction.

In another aspect, there is provided a method for agonizing the function of melanocortin receptors, in particular, melanocortin-4 receptors (MC4R), the method comprising a step for administering to a subject the above-described crystalline form V.

In another aspect, there is provided a method for treating obesity, diabetes, inflammation, or erectile dysfunction, the method comprising a step for administering to a subject the above-described crystalline form V.

### ADVANTAGEOUS EFFECTS

The crystalline form V, according to the present invention, exhibits excellent agonistic action on melanocortin receptors, in particular, melanocortin-4 receptors (MC4R), and thus can be usefully used for preventing or treating obesity, diabetes, inflammation, and erectile dysfunction.

The crystalline form V, according to the present invention, exhibits an on-target effect on melanocortin-4 receptors, thereby exhibiting weight loss and diet reduction effects, without affecting anxiety and depression. In addition, it can be administered without any safety issues, such as side effects of human ether-a-go-go related gene (hERG) inhibition or mutagenesis.

In addition, the crystalline form V, according to the present invention, has purity, yield, physical and chemical stability, which are more excellent than the crude compound of formula 1, the amorphous compound of formula 1, or any other crystalline forms of formula 1.

Specifically, the crystalline form V may have superior solubility, storage stability, and production stability to the compound of formula 1, the amorphous compound of formula 1, or any other crystalline forms of formula 1.

### BRIEF DESCRIPTION OF THE DRAWINGS

FIG. 1 is a graph of the XRD result of Preparation Example 4.
FIG. 2 is a graph of the DSC result of Preparation Example 4.
FIG. 3 is a graph of the TGA result of Preparation Example 4.
FIG. 4 is a graph of the XRD result of Example 1.
FIG. 5 is a graph of the DSC result of Example 1.
FIG. 6 is a graph of the TGA result of Example 1.
FIG. 7 is a graph of the XRD result of Examples 2 and 3.
FIG. 8 is a graph of the DSC result of Examples 2 and 3.
FIG. 9 is a graph of the TGA result of Examples 2 and 3.

### MODE FOR CARRYING OUT THE INVENTION

Hereinafter, the present invention will be described in more detail through Preparation Examples and Examples. However, these Examples are merely illustrative of the present invention, and the scope of the present invention is not limited thereto.

### Preparation Example 1: Preparation of methyl (2S,4S)-4-(N-((1s,4R)-4-methylcyclohexyl)isobutyramido)pyrrolidine-2-carboxylate hydrochloride

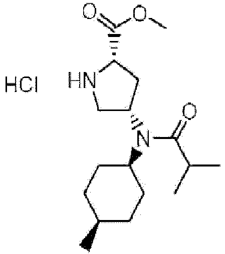

The title compound was obtained through the following steps A, B, C, D, and E.

### Step A: Preparation of 1-(tert-butyl) 2-methyl (2S,4S)-4-azidopyrrolidine-1,2-dicarboxylate

1-(tert-butyl) 2-methyl (2S,4R)-4-((methylsulfonyl)oxy)pyrrolidine-1,2-dicarboxylate (48.5 g, 150 mmol) was dissolved in N,N'-dimethylformamide (250 ml) under nitrogen, and sodium azide (19.5 g, 300 ml) was added. After stirring at 80°C for 16 hours, the reaction solvent was concentrated under reduced pressure, water was added, and extraction was performed twice with ethyl acetate. The organic layer was washed with an aqueous sodium chloride solution and water, dried over anhydrous magnesium sulfate, and filtered. The filtrate was concentrated under reduced pressure to obtain crude 1-(tert-butyl) 2-methyl (2S,4S)-4-azidopyrrolidine-1,2-dicarboxylate (39.59 g, 98%), which was used in the next step without purification.
MS [M+H] = 271 (M+1)
¹H NMR (400 MHz, CD3OD) δ 4.43-4.37 (m, 1H), 4.35-4.27 (br, 1H), 3.77 (s, 1.8H), 3.76 (s, 1.2H), 3.73-3.66 (m, 1H), 3.44-3.38 (m, 1H), 2.63-2.49 (m, 1H), 2.19-2.11 (m, 1H), 1.50 (s, 4.5H), 1.44 (s, 4.5H)

### Step B: Preparation of 1-(tert-butyl) 2-methyl (2S,4S)-4-aminopyrrolidine-1,2-dicarboxylate

1-(tert-butyl) 2-methyl (2S,4S)-4-azidopyrrolidine-1,2-dicarboxylate (24.59 g, 91.0 mmol) obtained in step A above was dissolved in tetrahydrofuran (180 ml), and 1 M trimethylphosphine tetrahydro solution (109.2 ml, 109.2 mmol) was slowly added at 0°C. The mixture was stirred at the same temperature for 1 hour and then at room temperature for 3 hours. After the reaction solvent was concentrated under reduced pressure, dichloromethane (100 ml) and water (150 ml) were added, and the mixture was stirred for about 30 minutes. The layers were separated and were extracted once more with dichloromethane, and the organic layer was dried over anhydrous magnesium sulfate and was filtered. The filtrate was concentrated under reduced pressure to obtain crude 1-(tert-butyl) 2-methyl (2S,4S)-4-aminopyrrolidine-1,2-dicarboxylate (20.62 g, 93%), which was used in the next step without purification.
MS [M+H] = 245 (M+1)
^{1H} NMR (400 MHz, CD3OD) δ 4.27 (m, 1H), 3.77 (s, 1.8H), 3.76 (s,1.2H), 3.75-3.67 (m, 1H), 3.50-3.42 (m, 1H), 3.22-3.17 (m, 1H), 2.58-2.47 (m,1H), 1.82-1.71 (m, 1H), 1.48 (s, 4.5H), 1.42 (s, 4.5H)

### Step C: Preparation of 1-(tert-butyl) 2-methyl (2S,4S)-4-(((1s,4R)-4-methylcyclohexyl)amino)pyrrolidine-1,2-dicarboxylate

1-(tert-butyl) 2-methyl (2S,4S)-4-aminopyrrolidine-1,2-dicarboxylate (20.62 g, 84.4 mmol) obtained in step B above was dissolved in dichloroethane (150 ml), and 4-methylcyclohexanone (9.5 ml, 101.3 mmol) was added. Sodium triacetoxyborohydride (26.8 g, 126.6 mmol) was added at 0°C, and the mixture was stirred at room temperature for 16 hours. The reaction solvent was concentrated under reduced pressure, water was added, and extraction was performed twice with ethyl acetate. The organic layer was washed with an aqueous sodium chloride solution, dried over anhydrous magnesium sulfate, and filtered. The filtrate was concentrated under reduced pressure and purified by column chromatography to obtain 1-(tert-butyl) 2-methyl (2S,4S)-4-(((1s,4R)-4-methylcyclohexyl)amino)pyrrolidine-1,2-dicarboxylate (22.9 g, 80%) .
MS [M+H] = 341 (M+1)
^{1H} NMR (400 MHz, CD3OD) δ 4.26 (m, 1H), 3.76 (s, 1.8H), 3.75 (s, 1.2H), 3.78-3.71 (m, 1H), 3.49-3.40 (m, 1H), 3.22-3.16 (m, 1H), 2.69-2.60(br, 1H), 2.58-2.46 (m, 1H), 1.87-1.77 (m, 1H), 1.73-1.63 (m, 1H), 1.62-1.35(m, 8H), 1.48 (s, 4.5H), 1.42 (s, 4.5H), 0.96 (d, 3H)

### Step D: Preparation of 1-(tert-butyl) 2-methyl (2S,4S)-4-(N-((1s,4R)-4-methylcyclohexyl)isobutyramido)pyrrolidine-1,2-dicarboxylate

1-(tert-butyl) 2-methyl (2S,4S)-4-(((1s,4R)-4-methylcyclohexyl)amino)pyrrolidine-1,2-dicarboxylate obtained in step C above (37.29 g, 109.5 mmol) was dissolved in dichloromethane (500 ml), triethyl amine (61.1 ml, 438.1 mmol) was added, and then isobutyryl chloride (11.7 ml, 219 mmol) was slowly added at 0°C. After the mixture was stirred at room temperature for 16 hours, the reaction solvent was concentrated under reduced pressure, an aqueous sodium hydrogen carbonate solution was added, and extraction was performed twice with ethyl acetate. The organic layer was washed with an aqueous sodium chloride solution and water, dried over anhydrous magnesium sulfate, and filtered. The filtrate was concentrated under reduced pressure, and purified by column chromatography to obtain 1-(tert-butyl) 2-methyl (2S,4S)-4-(N-((1s,4R)-4-methylcyclohexyl)isobutyramido)pyrrolidine-1,2-dicarboxylate (38.79 g, 86%).
MS [M+H] = 411 (M+1)
^{1H} NMR (400 MHz, CD3OD) δ 4.27 (m, 1H), 3.76 (s, 1.8H), 3.75 (s, 1.2H), 3.78-3.72 (m, 1H), 3.50-3.41 (m, 1H), 3.33-3.14 (m, 1H), 2.69-2.60 (m, 2H), 2.57-2.43 (m, 1H), 1.87-1.79 (m, 1H), 1.70-1.61 (m, 1H), 1.60-1.32 (m, 8H), 1.47 (s, 4.5H), 1.41 (s, 4.5H), 1.10 (dd, 6H), 0.99 (d, 3H)

### Step E: Preparation of methyl (2S,4S)-4-(N-((ls,4R)-4-methylcyclohexyl)isobutyramido)pyrrolidine-2-carboxylate hydrochloride

1-(tert-butyl) 2-methyl (2S,4S)-4-(N-((1s,4R)-4-methylcyclohexyl)isobutyramido)pyrrolidine-1,2-dicarboxylate (34.0 g, 82.8 mmol) obtained in step D above was dissolved in dichloromethane (200 ml), and a solution of 4 N hydrochloric acid in 1,4-dioxane solution (82.8 ml, 331.3 mmol) was added at 0°C. After the mixture was stirred at room temperature for 6 hours, the reaction solvent was concentrated under reduced pressure to obtain crude (28.7 g, 99%), which was used in the next step without purification.
MS[M+H] = 311 (M+1)

### Preparation Example 2: Preparation of (3S,4R)-1-(tert-butyl)-4-(4-chlorophenyl)pyrrolidine-3-carboxylic acid

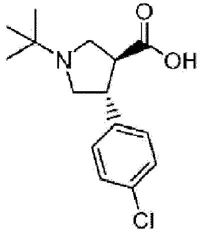

The title compound was obtained according to the method described in international patent publication no. WO 2004/092126.
MS[ M+H] = 282 (M+1)
^{1H} NMR (400 MHz, CD3OD) δ 7.43-7.33 (m, 4H), 3.90-3.69 (m, 3H), 3.59 (dd, J = 11.2, 10.0 Hz, 1H), 3.29 (dd, J = 11.2, 11.2 Hz, 1H), 3.18-3.09 (m, 1H), 1.44 (s, 9H)

### Preparation Example 3: Preparation of N-((3S,5S)-1-((3S,4R)-1-(tert-butyl)-4-(4-chlorophenyl)

### pyrrolidine-3-carbonyl)-5-(morpholine-4-carbonyl)pyrrolidin-3-yl)-N-((1s,4R)-4-methylcyclohexyl)isobutyramide

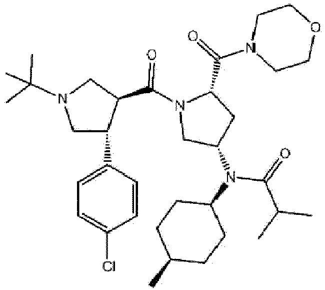

The title compound was obtained through the following steps A, B, and C.

### Step A: Preparation of methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate

Methyl (2S,4S)-4-(N-((1s,4R)-4-methylcyclohexyl)isobutyramido)pyrrolidine-2-carboxylate hydrochloride (28.7 g, 82.73 mmol) obtained in Preparation Example 1, (3S,4R)-1-(tert-butyl)-4-(4-chlorophenyl)pyrrolidine-3-carboxylic acid (24.5 g, 86.87 mmol) obtained in Preparation Example 2, 1-(3-dimethylaminopropyl)-3-ethylcarbodiimide hydrochloride (22.2 g, 115.83 mmol), and 1-hydroxybenzotriazole hydrate (15.7 g, 115.83 mmol) were dissolved in N,N'-dimethylformamide (400 ml), and N,N'-diisopropylethylamine (72.0 ml, 413.66 mmol) was added slowly. The mixture was stirred at room temperature for 16 hours, the reaction solvent was concentrated under reduced pressure, 0.5 N sodium hydroxide aqueous solution was added, and then, extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (41.19 g, 87%).
MS [M+H] = 575 (M+1)

### Step B: Preparation of (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid

Methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (39.4 g, 68.62 mmol) obtained in step A above was dissolved in methanol (450 ml), and then, 6 N sodium hydroxide aqueous solution (57.2 ml, 343.09 mmol) was added. The mixture was stirred at room temperature for 16 hours, the pH was adjusted to about 5 with 6 N aqueous hydrochloric acid solution, and then the reaction solution was concentrated under reduced pressure. The concentrate was dissolved in dichloromethane, and then the insoluble solid was filtered through a paper filter. The filtrate was concentrated under reduced pressure to obtain the crude title compound (38.4 g, 99%), which was used in the next step without purification.
MS [M+H] = 561 (M+1)

### Step C: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

(2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid (38.4 g, 68.60 mmol) obtained in step B above, 1-(3-dimethylaminopropyl)-3-ethylcarbodiimide hydrochloride (18.4 g, 96.04 mmol), and 1-hydroxybenzotriazole hydrate (13.0 g, 96.04 mmol) were dissolved in N,N'-dimethylformamide (200 ml), and then morpholine (5.9 ml, 68.80 mmol) and N,N'-diisopropylethylamine (59.7 ml, 343.02 mmol) were sequentially and slowly added. The mixture was stirred at room temperature for 16 hours, the reaction solution was concentrated under reduced pressure, 0.5 N sodium hydroxide aqueous solution was added, and then extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide (37.05 g, 86%).
MS [M+H] = 630 (M+1)

### Preparation Example 4: Preparation of amorphous compound of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

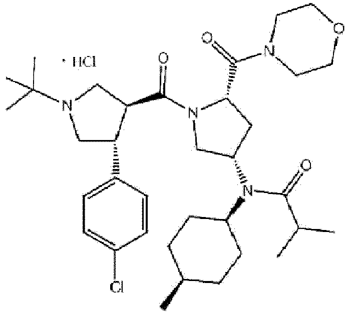

Based on 1 g of the compound (MC70) prepared in Preparation Example 3 above, 19 mL of MBTE was used to dissolve the compound (MC70) at 25°C. After dissolution was completed, 1 mL of heptane was added, and then the mixture was cooled to -5 to 0°C. After the set temperature was reached, 1 equivalent of 4 M HCl/EtOAc was added dropwise, the mixture was stirred for about 90 minutes, and filtered to obtain the title compound (MC71). (yield: about 90%)

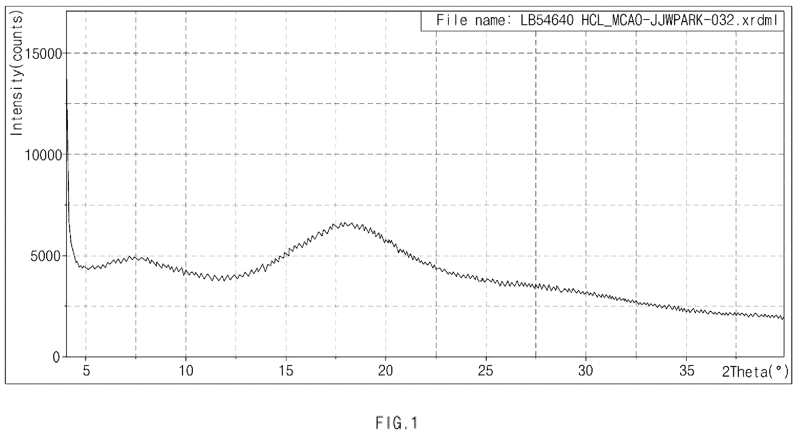

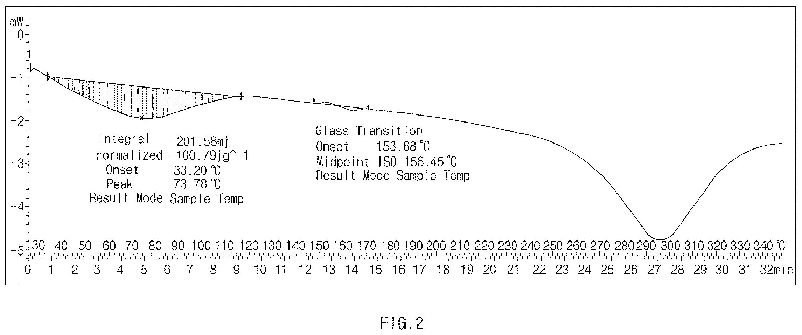

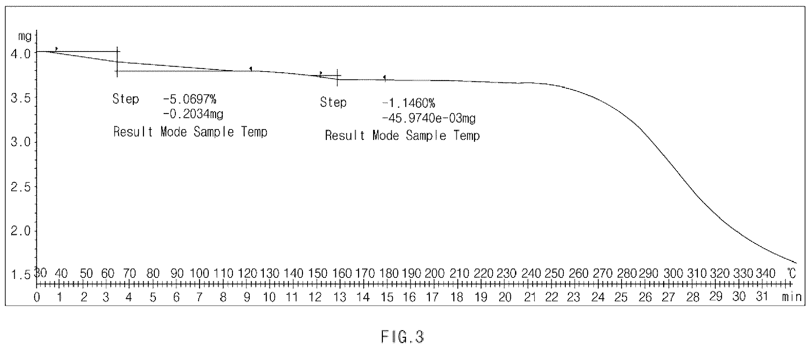

The XRD (FIG. 1), DSC (FIG. 2), and TGA (FIG. 3) analyses results for the compound of Preparation Example 4 were shown in FIGs. 1 to 3, respectively. The analyses results confirmed that it was an amorphous compound. The XRD, DSC, and TGA analysis methods each are as described in Experimental Examples for Example 1 below.

### Example 1.

### Preparation of crystalline form V of solvate with anisole of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

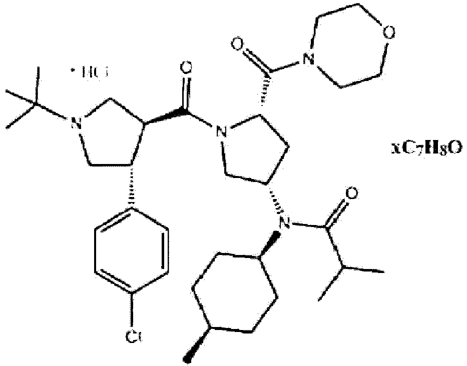

The compound of Preparation Example 4 (MC71) prepared above was dissolved at 20 to 25°C in a crystallization solvent to prepare a mixed solution of concentration of 100 mg/mL. Herein, the crystallization solvent was a mixed solvent in which anisole and diethyl ether were mixed in a volume ratio of 1:3. After dissolution was completed, the mixture was stored at room temperature for 24 hours and then filtered to obtain the title crystalline form V of the solvate with anisole.

### Example 2.

### Preparation of crystalline form V of solvate with anisole of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

The crystals obtained in Example 1 were washed with diethyl ether and then stored in a circulating oven at 60°C for 8 hours to obtain the title crystalline form V of the solvate with anisole.

### Example 3.

### Preparation of crystalline form V of solvate with anisole of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

The title crystalline form V of the solvate with anisole was obtained in the same manner as in Example 2, except that the crystals obtained in Example 1 were washed with diethyl ether and then stored at room temperature of 20 to 25°C for 30 days.

### Comparative Example 1

1 g of the compound (MC71) of Preparation Example 4 prepared above was dissolved in 1 mL of EtOAc at room temperature. After completion of dissolution, the mixture was stirred for about 43 hours with an electronic stirrer, but the same crystals as in Examples 1 to 3 were not obtained.

### Experimental Example 1. Evaluation of the crystalline form of Example 1

### [Experimental example 1-1. XRD assessment]

For powder XRD diffraction pattern, PXRD (Cu Kα radiation (λ=1.54 Å), Rigaku, Miniflex 600, Japan) was used, and the tube voltage and tube current were set to 45 kV and 15 mA, respectively, and then Bragg angles (2Θ) in a range of 3 to 40° were measured with the scan rate of 0.02°/s and the step speed of 4°/min.

The XRD measurement result of the crystalline form V obtained in Example 1 is shown in FIG. 4.

As confirmed in the spectrum shown in FIG. 4, the crystalline form V, according to Example 1, exhibited characteristic peaks (2Θ) at 4.42°, 7.84°, 12.58°, 16.20°, 20.08°, and 23.65°. The specific values of the XRD are shown in Table 1 below.

**[Table 1]**

| 2θ | Relative Intensity (I/I₀) |
|---|---|
| 4.42 | 1947 |
| 7.84 | 1374 |
| 12.58 | 1122 |
| 16.20 | 965 |
| 20.08 | 1709 |
| 23.65 | 1448 |

[Experimental example 1-2. Differential scanning calorimetry (DSC)] DSC (Q2000, TA instrument, USA) was used to measure de-solvated temperature, transient temperature, and melting temperature. 30°C to 300°C was scanned at a rate of 10°C/min with a nitrogen purging at a rate of 50 ml/min.

The DSC measurement result of the crystalline form V obtained in Example 1 is shown in FIG. 5.

As can be seen in FIG. 5, for the crystalline form V, according to Example 1, an endothermic peak was observed at about 126.2°C. The temperature values have an error of ±5°C.

### [Experimental example 1-3. Thermogravimetric Analysis (TGA)J

Volatile content analysis including surface water and the solvate was performed by Thermogravimetric analysis (TGA8000, PerkinElmer, USA). 5 mg of a sample was weighed in an open aluminum pan, and TGA was performed. The sample was heated from 30°C to 300°C at a rate of 10°C/min. During measurement, nitrogen gas was introduced into the inside of the instrument at a rate of 20 mL/min to block the inflow of oxygen and other gases.

The TGA measurement result of the crystalline form V obtained in Example 1 is shown in FIG. 6.

As can be seen in FIG. 6, for the crystalline form V, according to Example 1, a weight loss of about 11.8% was observed at a temperature of 120°C to 130°C. After about 250°C, a weight loss due to decomposition occurred. The temperature values have an error of ±5°C.

### Experimental Example 2. Evaluation of the crystalline forms of Examples 2 and 3

### [Experimental example 2-1. XRD assessment]

The XRD measurement result of the crystalline forms V of Examples 2 and 3 obtained above in the same manner as in Experimental Example 1-1 is shown in FIG. 7.

As confirmed in the spectrum shown in FIG. 7, all the crystalline forms V, according to Examples 2 and 3, exhibited characteristic peaks (2θ) at 4.92°, 7.20°, 9.22°, 12.22°, 16.48°, 17.72°, and 20.24°. The specific values of the XRD are shown in Table 2 below.

**[Table 2]**

| 2θ | Relative Intensity (I/I₀) | |
|---|---|---|
| | Example 2 | Example 3 |
| 4.92 | 2752 | 1690 |
| 7.20 | 1663 | 1630 |
| 9.22 | 947 | 1311 |
| 12.22 | 745 | 889 |
| 16.48 | 1164 | 1667 |
| 17.72 | 647 | 648 |
| 20.24 | 1021 | 1343 |

[Experimental example 2-2. Differential scanning calorimetry (DSC)J The DSC measurement result of the crystalline forms V of Examples 2 and 3 obtained above in the same manner as in Experimental Example 1-2 is shown in FIG. 8.

As can be seen in FIG. 8, for the crystalline forms V of Examples 2 and 3, an endothermic peak was observed at 192°C to 194°C. The temperature values have an error of ±5°C.

### [Experimental example 2-3. Thermogravimetric Analysis (TGA)]

The TGA analysis result of the crystalline forms V of Examples 2 and 3 obtained above in the same manner as in Experimental Example 1-3 is shown in FIG. 9.

As can be seen in FIG. 9, for the crystalline forms V, a weight loss of about 1% to 3% was observed at a temperature of 120 to 130°C. After about 250°C, a weight loss due to decomposition occurred. The temperature values have an error of ±5°C.

## Claims

1. A crystalline form V of a compound of the following formula 1, a pharmaceutically acceptable salt thereof, or a solvate thereof,
wherein the X-ray powder diffraction pattern has 3 or more characteristic peaks selected from among peaks with the following diffraction angles (2Θ values) of: 4.42±0.2°, 4.92±0.2°, 7.20±0.2°, 7.84±0.2°, 9.22±0.2°, 12.22±0.2°, 12.58±0.2°, 16.20±0.2°, 16.48±0.2°, 17.72±0.2°, 20.24±0.2°, 20.08±0.2°, and 23.65±0.2°, 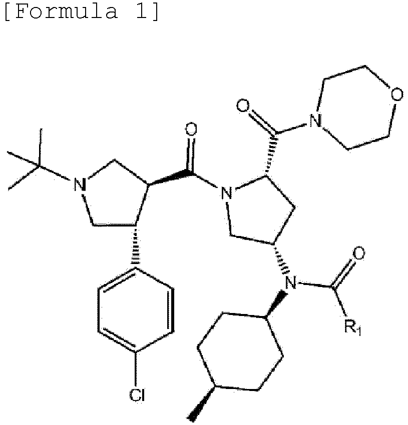 wherein
R₁ is a C₂-C₅ alkyl.

2. The crystalline form V of claim 1, wherein the X-ray powder diffraction pattern has characteristic peaks including the following diffraction angles (2Θ values): 4.42±0.2°, 7.84±0.2°, 12.58±0.2°, 16.20±0.2°, 20.08±0.2°, and 23.65±0.2°.

3. The crystalline form V of claim 1, wherein the X-ray powder diffraction pattern has characteristic peaks including the following diffraction angles (2Θ values): 4.92±0.2°, 7.20±0.2°, 9.22±0.2°, 12.22±0.2°, 16.48±0.2°, 17.72±0.2°, and 20.24±0.2°.

4. The crystalline form V of claim 1, wherein the pharmaceutically acceptable salt of the compound of the formula 1 is selected from the group consisting of hydrochloride, sulfate, nitrate, phosphate, hydrobromide, and hydroiodide of the compound.

5. The crystalline form V of claim 1, which is a crystalline form of a solvate of hydrochloride of the compound of the formula 1.

6. The crystalline form V of claim 5, wherein the solvate is a solvate with an aromatic ether.

7. The crystalline form V of claim 6, wherein the solvate is a solvate with methyl phenyl ether.

8. A method for preparing the crystalline form V described in any one of claims 1 to 7, the method comprising the steps of:
preparing a mixed solution by dissolving the compound of the formula 1 in a crystallization solvent containing an aromatic ether; and
obtaining crystals from the mixed solution.

9. The method for preparing the crystalline form V of claim 8, wherein the crystallization solvent further includes an aliphatic aprotic organic solvent.

10. The method for preparing the crystalline form V of claim 8, wherein the aromatic ether includes methyl phenyl ether, ethyl phenyl ether, diphenyl ether, or a mixture thereof.

11. The method for preparing the crystalline form V of claim 9, wherein the aliphatic aprotic organic solvent includes diethyl ether, pentane, hexane, heptane, cyclohexane, or a mixture thereof.

12. The method for preparing the crystalline form V of claim 9, wherein, as the crystallization solvent, the aromatic ether and the aliphatic aprotic organic solvent are used in a volume ratio of 1:1 to 1:10.

13. The method for preparing the crystalline form V of claim 8, further comprising a step for washing the obtained crystals with an aliphatic ether solvent.

14. The method for preparing the crystalline form V of claim 13, wherein the crystallization solvent further includes an aliphatic aprotic organic solvent, and
the aliphatic aprotic organic solvent included in the crystallization solvent is the same as or different from the aliphatic ether solvent used in the washing step.

15. The method for preparing the crystalline form V of claim 14, wherein the crystallization solvent includes methyl phenyl ether and diethyl ether, and
the washing solvent includes diethyl ether.

16. The method for preparing the crystalline form VI of claim 13, wherein the washing step includes washing the obtained crystals with an aliphatic aprotic organic solvent at least once, and then obtaining crystals at 0°C to 200°C.

17. A pharmaceutical composition comprising the crystalline form V, according to any one of claims 1-7, and a pharmaceutically acceptable carrier.

18. A pharmaceutical composition for agonizing the function of a melanocortin-4 receptor, the composition comprising the crystalline form V, according to any one of claims 1-7, and a pharmaceutically acceptable carrier.

19. The pharmaceutical composition of claim 18, which is for preventing or treating obesity, diabetes, inflammation, or erectile dysfunction.
